# FASCICULE DE BREVET EUROPEEN

(11) **EP 2 961 481 B1**
(45) Date de publication et mention de la délivrance du brevet: **28.06.2017**
(21) Numéro de dépôt: 14718638.1
(22) Date de dépôt: 24.02.2014
(51) Int. Cl.: A61P 31/00, A61K 38/17, A61K 45/06, A61K 31/728, A61K 38/08, A61K 9/00, A61K 9/06, A61K 47/36

(54) **COMPOSITION DERMATOLOGIQUE ANTIMICROBIENNE TOPIQUE**
TOPISCHE ANTIMIKROBIELLE DERMATOLOGISCHE ZUBEREITUNG
TOPICAL ANTIMICROBIAL DERMATOLOGICAL COMPOSITION

(30) Priorité: 28.02.2013 FR 1351766
(43) Date de publication de la demande: 06.01.2016
(60) Demande divisionnaire: 17163754.9
(73) Titulaire: Laboratoires Thea S.A.S., 63100 Clermont-Ferrand (FR)
(72) Inventeur: LEFEVRE, Jean-Marie, F-80000 Amiens (FR); PEYROT, Jacques, F-63000 Clermont-Ferrand (FR); ALLART, Jean-Claude, décédé (FR)
(74) Mandataire: L'Helgoualch, Jean
(86) Numéro de dépôt international: PCT/FR2014/050383
(87) Numéro de publication internationale: WO 2014/131974

(56) Documents cités:
- WO-A1-2006/130974
- WO-A2-2010/081800
- US-A1- 2009 258 045
- DATABASE GNPD [Online] MINTEL; janvier 2013 (2013-01), Marie Veronique Organics: "Night Serum with Retinol", XP002724257, Database accession no. 1947053
- TOPPAZZINI M ET AL: "Can the interaction between the antimicrobial peptide LL-37 and alginate be exploited for the formulation of new biomaterials with antimicrobial properties?", CARBOHYDRATE POLYMERS, APPLIED SCIENCE PUBLISHERS, LTD. BARKING, GB, vol. 83, no. 2, 10 janvier 2011 (2011-01-10), pages 578-585, XP027488135, ISSN: 0144-8617, DOI: 10.1016/J.CARBPOL.2010.08.020 [extrait le 2010-08-18]
- MALINA AMIR ET AL: "CONJUGATION OF FATTY ACIDS WITH DIFFERENT LENGTHS MODULATES THE ANTIBACTERIAL AND ANTIFUNGAL ACTIVITY OF A CATIONIC BIOLOGICALLY INACTIVE PEPTIDE", BIOCHEMICAL JOURNAL, PUBLISHED BY PORTLAND PRESS ON BEHALF OF THE BIOCHEMICAL SOCIETY, vol. 390, no. PT 3, 15 septembre 2005 (2005-09-15), pages 695-702, XP009084335, ISSN: 0264-6021
- "beta-defensin 2 release by human keratinocytes following low molecular weight hyaluronan fragments treatment", JOURNAL OF THE AMERICAN ACADEMY OF DERMATOLOGY, MOSBY, INC, US, vol. 58, no. 2, 1 février 2008 (2008-02-01), page AB29, XP022456908, ISSN: 0190-9622
- "Hyaluronan-induced beta-defensin 2 production in murine skin", JOURNAL OF THE AMERICAN ACADEMY OF DERMATOLOGY, MOSBY, INC, US, vol. 58, no. 2, 1 février 2008 (2008-02-01), page AB15, XP022456853, ISSN: 0190-9622

## Description

La présente invention concerne une nouvelle composition utile en thérapeutique, présentant des propriétés antimicrobiennes, et plus particulièrement une nouvelle composition à base d'acide hyaluronique et de peptide antimicrobien cationique, utile en médecine humaine et vétérinaire, notamment en dermatologie et en ophtalmologie pour le traitement des infections microbiennes.

La peau constitue à la fois une barrière anatomique vivante et une zone d'échange entre le corps et son environnement, dont l'efficacité conditionne le maintien d'un bon équilibre homéostasique. La peau est un véritable organe comprenant plusieurs couches intégrées, allant de la couche superficielle, l'épiderme, jusqu'aux couches plus profondes, le derme et l'hypoderme, où chacune de ces couches remplit des fonctions permettant à l'ensemble de réagir et de s'adapter à son environnement.

L'épiderme, principalement composé de kératinocytes, de mélanocytes et des cellules de Langerhans, a une épaisseur variable selon les différentes parties du corps, et constitue la couche externe de la peau pour assurer la protection du corps vis-à-vis de son environnement extérieur. Le derme est la couche la plus épaisse, traversée par des fibres nerveuses et des vaisseaux sanguins, se composant principalement de collagène, d'élastine, de protéoglycanes et de glycosaminoglycanes, principalement synthétisés par les fibroblastes dermiques. Les fibres de collagène assurent la résistance mécanique et la texture de la peau, l'élastine est responsable de son élasticité, les glycosaminoglycanes et les protéoglycanes jouent un rôle majeur de structure et d'hydratation de la peau. La couche la plus profonde de la peau forme l'hypoderme, contenant les adipocytes qui produisent des lipides assurant la formation d'une couche grasse protégeant les muscles, les os et les organes internes contre les chocs.

Les changements dans la structure de l'épiderme, tels qu'une augmentation de l'humidité ou l'existence d'irritations ou de plaies cutanées d'origines diverses, ou l'évolution d'une dermatose, favorisent la colonisation et l'infection de la peau par les micro-organismes pathogènes. La pullulation microbienne qui en résulte a pour effet de modifier les conditions de la cicatrisation en la retardant ou en la bloquant, et en favorisant la diffusion d'une infection à partir de la plaie initiale.

Ainsi, la peau est constamment attaquée par des microorganismes pathogènes, mais la couche cornée de l'épiderme, en raison de son pH, de sa teneur en eau relativement faible et de la présence de peptides antibactériens ayant une action bactéricide, joue le rôle de première défense antimicrobienne contre ces micro-organismes pathogènes.

La plupart des colonies bactériennes se transforment en biofilms, structure membranaire de survie microbienne, détournant les facteurs de croissance et de cicatrisation en créant une inflammation qui empêche le processus de défense antimicrobienne d'agir efficacement. En raison de l'utilisation très répandue, et parfois excessive, d'antibiotiques, les bactéries ont développé une résistance aux antibiotiques qui limitent et même annulent leurs effets antibactériens. Les bactéries des biofilms résistent à la plupart des antibiotiques et des antiseptiques, et il est donc nécessaire de mettre au point des compositions susceptibles de lutter efficacement contre les micro-organismes pathogènes respectant le plus possible l'homéostasie cutanée, et ne favorisant pas le développement de souches résistantes, dangereuses pour l'homme et son environnement.

On sait que les peptides antimicrobiens cationiques ont une action sur la membrane des bactéries par liaison électrostatique entre le peptide cationique et les phospholipides chargés négativement se trouvant dans la structure externe de la membrane des bactéries Gram négatif, tandis que chez les bactéries Gram positif, la liaison se fait entre le peptide et les composants du peptidoglycane autour de la membrane plasmique des bactéries. Le caractère cationique des peptides antimicrobiens explique leur affinité pour la membrane anionique des bactéries, et la fixation est d'autant plus facile que la charge est importante. Après fixation, les peptides modifient la perméabilité membranaire, formant des pores qui provoquent l'osmolyse puis la mort de la bactérie.

Ainsi les peptides antimicrobiens, en particulier les peptides cationiques, agissent au niveau cutané en protégeant la peau contre les infections bactériennes et l'inflammation.

Les défensines constituent une famille de peptides antimicrobiens naturels impliqués dans l'immunité non spécifique, ou innée. Ce sont de petits peptides cationiques antimicrobiens constitués de chaînes de 36 à 42 acides aminés comportant des ponts disulfures intramoléculaires. Chez l'homme, elles possèdent un large spectre d'activités antibactérienne et antifongique très efficaces, et sont divisées en deux groupes, à savoir les α-défensines et les β-défensines. Ces dernières sont présentes sur l'ensemble des epithelia, dont l'epithelium cutané et la muqueuse lacrymale et buccale, et au sein de nombreux organes, et jouent un rôle important dans les réponses aux infections. Les β-défensines sont libérées suite à l'activation de récepteurs spécifiques, les Toll Like Receptors (TLR).

Des études ont montré que la flore bactérienne commensale et la flore pathogène utilisent différentes voies d'induction des β-défensines. Ainsi, la peau dispose de moyens moléculaires pour les différencier et lutter contre les bactéries.

Les "Toll Like Receptors" (TLR) sont des récepteurs membranaires exprimés par la famille de gènes TLR au rôle fondamental dans la reconnaissance de germes pathogènes et l'activation de l'immunité innée. Ils modulent la production de cytokines nécessaires à une immunité efficace. Les TLR4 et TLR2 sont actifs lors de la reconnaissance des lipopolysaccharides (LPS) présents sur les bactéries Gram-négatif. Le LPS est une endotoxine bactérienne pro-inflammatoire. Le TLR2 reconnaît plus particulièrement le peptidoglycane des bactéries Gram-positif et le TLR4 reconnaît le LPS des bactéries Gram-négatif. Dans la peau, les protéines TLR2 et TLR4 sont exprimées sur les kératinocytes et TLR2 sur les glandes sébacées.

Les défensines jouent le rôle original d'éliciteurs, c'est-à-dire de molécule susceptible de déclencher un mécanisme de défense de l'organisme. Ainsi naturellement présentes dans la peau, elles sont stimulées pour renforcer la protection des peaux fragilisées. Dans un environnement qui est souvent hostile, il est important d'aider les fonctions biologiques pour maintenir la qualité et l'état d'une peau belle et saine. La notion d'éliciteur implique d'inciter les voies biologiques présentes dans la peau à mieux réagir face aux agressions externes

Les propriétés antimicrobiennes de la β-défensine humaine (HBD-3) sont décrites dans la demande de brevet WO 0192309.

L'acide hyaluronique est un polymère naturel à base de disaccharide comprenant des unités acide D-glucuronique et N-acétylglucosamine liées par liaison glucosidique. Ses propriétés sont très variables selon son poids moléculaire. Les acides hyaluroniques à haut poids moléculaire, supérieur à 1.000 kDa, sont essentiellement utilisés pour favoriser l'hydratation de la peau grâce au réseau glucidique hydrophile qu'ils constituent. Les acides hyaluroniques de faible poids moléculaire, inférieur à 50 kDa environ, peuvent franchir la barrière du stratum corneum et stimulent les récepteurs CD44 responsables de la néosynthèse de l'acide hyaluronique dans le derme.

L'acide hyaluronique, comme le collagène, est un des constituants principaux de la matrice extracellulaire du derme et il joue un rôle important dans la croissance cellulaire et dans le maintien de l'hydratation. Au cours du vieillissement de la peau, on observe une diminution de sa concentration dans le derme. L'acide hyaluronique est un glycosaminoglycane souvent utilisé dans des compositions cosmétiques et dermatologiques, généralement sous forme de hyaluronate de sodium, notamment pour favoriser l'hydratation, et pour stimuler la cicatrisation et les défenses naturelles de la peau. Il est également utilisé en chirurgie esthétique pour le comblement des rides, dans des traitements médicaux contre l'arthrose, et en ophtalmologie. Divers dérivés d'acide hyaluronique, réticulés ou non réticulés, susceptibles de présenter une bonne résistance à la dégradation enzymatique, et utilisables en particulier dans des compositions cosmétiques, sont décrits par exemple dans la demande WO 2011080450. Diverses formes d'acide hyaluronique sont bien connues et disponibles dans le commerce.

On a proposé d'associer l'acide hyaluronique ou des dérivés avec le chondroïtine sulfate, comme dans la demande WO 2009073437, ou avec un rétinoïde et un oligosaccharide comme dans le brevet FR 2.894.827. Des sels de métaux lourds d'acide hyaluronique ont aussi été proposés dans des compositions antimicrobiennes, comme décrit dans la demande WO 8705517. Le brevet US6.180.601 décrit une composition pharmaceutique utilisant un acide hyaluronique de faible poids moléculaire, c'est-à-dire 50 à 200 kDa, formant une matrice incorporant un peptide ou une protéine, par exemple une hormone de croissance. La demande WO 2006130974 concerne des peptides contenant de préférence 15 à 40 amino-acides, pouvant se lier d'une part à l'acide hyaluronique et d'autre part à la capsule bactérienne contenant de l'acide hyaluronique, et pouvant être utilisé en présence d'un lipide pour former des liposomes. Une composition pharmaceutique comprenant un peptide dérivé de lactoferrine et de l'acide hyaluronique est décrite dans la demande WO 2010081800.

Les études effectuées par la demanderesse ont permis de montrer qu'il est possible d'obtenir une activité antimicrobienne efficace, équivalente à celle des antiseptiques et antibiotiques locaux, en associant un peptide cationique antimicrobien à un acide hyaluronique de poids moléculaire moyen et que l'activité antimicrobienne du peptide cationique se trouve potentialisée par l'association avec l'acide hyaluronique qui stimule la production de β-défensines. Par activité antimicrobienne, on entend une activité antibactérienne et/ou antivirale et/ou antifongique.

L'objet de la présente invention est de proposer une nouvelle composition topique présentant des propriétés antimicrobiennes permettant de traiter efficacement les infections microbiennes cutanées et de renforcer les défenses naturelles de la peau, sans recourir à des antibiotiques ou à des antiseptiques chimiques.

La présente invention a donc pour objet une composition dermatologique, comprenant en association au moins un peptide cationique antimicrobien et de l'acide hyaluronique de poids moléculaire moyen ou un de ses sels, ladite composition étant telle que définie dans la revendication 1.

La présente invention a encore pour objet une composition dermatologique topique antimicrobienne, comprenant en association au moins un peptide cationique antimicrobien couplé à un lipide, et de l'acide hyaluronique, ou un de ses sels, de poids moléculaire compris entre 100 kDa et 800 kDa.

Enfin, la présente invention a pour objet une composition comprenant au moins un peptide cationique antimicrobien couplé à un lipide, et de l'acide hyaluronique de poids moléculaire moyen ou un de ses sels, en association, pour utilisation dans le traitement des infections microbiennes cutanées, phanériennes ou muqueuses.

Une telle composition est utile en dermatologie humaine pour le traitement de plaies cutanées grâce à ses propriétés antimicrobiennes.

Elle est aussi utile en dermatologie et en ophtalmologie, plus particulièrement pour le traitement des infections liées à des plaies aiguës chroniques, des folliculites, des surinfections des maladies inflammatoires dermatologiques, des blépharites, des meibonites et des conjonctivites.

Suivant la présente invention, le peptide antimicrobien cationique est de préférence un peptide comportant moins de 50 acides aminés, plus préférentiellement entre 3 et 30 acides aminés, et présentant un large spectre antimicrobien contre les bactéries Gram-positif et Gram-négatif.

On peut utiliser par exemple un peptide antimicrobien cationique choisi parmi les peptides linéaires à structure en hélice, les peptides comportant un ou plusieurs ponts disulfure et les peptides linéaires riches en certains acides aminés.

Le peptide cationique antimicrobien de l'invention comprenant moins de 50, de préférence moins de 30, amino-acides peut être choisi par exemple parmi des dérivés de la magainine, de la protégrine, de l'indolicidine et de l'histatine. On peut utiliser aussi un peptide cationique synthétique comme le pentachlorhydrate d'omiganan, analogue de l'indolicidine, le chlorhydrate d'iseganan, une protégrine synthétique, et l'acétate de pexiganan, analogue de la magainine. Ces peptides sont couplés par liaison covalente avec un lipide, et de préférence avec un acide gras tel que l'acide palmitique. Plus particulièrement, cette liaison covalente n'est pas liposomiale. On peut aussi utiliser un peptide disponible dans le commerce comme Oligopeptide-10 (Grant Industries) ou le Shield Bact Peptide (Infinitec) qui est un hexapeptide couplé à l'acide palmitique comprenant des ponts disulfure.

L'activité antimicrobienne des compositions suivant l'invention a été mise en évidence par des études in vitro et ex vivo comme indiqué plus loin, démontrant une triple action consistant à neutraliser les endotoxines microbiennes pour minimiser la sollicitation des Toll Like Receptors, mimer l'action des β-défensines pour procurer un effet anti-infectieux rapide, et moduler la réaction inflammatoire.

Le peptide antimicrobien doit être de préférence amphiphile. Ce caractère amphiphile est renforcé en couplant le peptide cationique avec un lipide, et notamment un acide gras linéaire ou ramifié, saturé ou insaturé, comportant de préférence 6 à 22 atomes de carbone, tel que l'acide oléique, l'acide linoléique, l'acide laurique, l'acide sapiénique, l'acide stéarique et l'acide palmitique. Le couplage de l'acide gras avec le peptide se fait de préférence par liaison covalente avec l'un au moins des acides aminés constituant le peptide. La liaison covalente peut être par exemple une liaison amide sur l'extrémité N-terminale du peptide. Les études effectuées ont montré que le caractère amphiphile ainsi renforcé favorise la fixation du peptide cationique sur la surface des bactéries et a pour effet
- une perturbation structurelle de la couche externe de la membrane bactérienne ;
- une accumulation d'acide gras sous forme de micelles, dans l'interface membranaire ;
- une perforation de cette double membrane par création de canaux ioniques.

Cette triple action a pour conséquence une lyse bactérienne liée à la perte d'imperméabilité de sa membrane et à l'action du peptide cationique sur les cibles anioniques intracytoplasmiques.

De plus, le peptide cationique antimicrobien de l'invention montre :
- un large spectre d'activité couvrant les bactéries Gram-négatif et Gram-positif, les infections mycosiques, virales et parasitaires,
- une grande rapidité d'action liée aux multiples cibles atteintes par l'acide gras, au sein de la bactérie.
- un risque très faible d'apparition de résistance comparé à celui observé avec les antibiotiques conventionnels.

Les études effectuées ont aussi montré que le peptide cationique antibactérien utilisé dans l'invention est capable de neutraliser la réponse des cellules hôtes aux endotoxines bactériennes, quel qu'en soit le type, selon les bactéries (Gram-négatif ou Gram-positif) qui les produisent. Il module ainsi la réponse immunitaire innée par la limitation de l'activité des TLR et la production du TNF α, ce qui tempère la cascade inflammatoire et l'orage cytokinique qui en découle (IL-6, IL-1b, IL-1a).

Il a aussi d'autres propriétés en relation avec l'immunité innée par l'action chimiotactique sur les monocytes et polynucléaires neutrophiles, par la libération d'histamine par les monocytes, et par l'inhibition des protéases limitant les destructions tissulaires et stimulant la réparation tissulaire.

Les études effectuées ont montré que ce mécanisme de défense de l'immunité innée en présence d'une attaque bactérienne, peut être optimisé par l'association du peptide cationique antimicrobien, de préférence lié à un acide gras, avec l'acide hyaluronique de poids moléculaire moyen. L'action immédiate sur les germes est complétée par la neutralisation des endotoxines qu'ils secrètent.

Il en résulte que la libération des cytokines pro inflammatoires est réduite et que l'action propre de l'acide hyaluronique peut alors s'exprimer pleinement par une fixation accélérée sur les Toll-Like Receptors et une sécrétion accrue de β-défensines, créant ainsi une deuxième ligne de défense contre les germes. Parallèlement les mécanismes de réparation sont stimulés pour restaurer rapidement l'effet barrière de l'épiderme.

Suivant la présente description, par acide hyaluronique, on entend l'acide hyaluronique sous forme libre ou d'un de ses sels de métaux alcalins et alcalino-terreux, par exemple le hyaluronate de sodium, de potassium, de calcium ou de magnésium, de poids moléculaire moyen, c'est-à-dire compris de préférence entre 100 et 800 kDa, plus préférentiellement entre 200 et 600 kDa.

L'acide hyaluronique utilisable dans l'invention est disponible dans le commerce sous diverses formes adaptées selon les utilisations envisagées. Il peut être produit industriellement en quantités importantes par extraction à partir de tissus animaux tels que des crêtes de coq, ou par fermentation bactérienne, ou encore par procédé biotechnologique à partir de substances végétales, par exemple du blé.

On peut utiliser par exemple de l'acide hyaluronique hydrolysé ou du sel de sodium d'acide hyaluronique, de poids moléculaire compris entre 200 et 600 kDa, comme ceux disponibles dans le commerce sous la marque PrimalHyal 300 (Soliance) de poids moléculaire proche de 300 kDa ou PrimalHyal 450 de poids moléculaire voisin de 450 kDa.

Les compositions suivant la présente invention peuvent comprendre une quantité efficace de chacun des actifs ci-dessus, et par exemple entre 0,05 et 2% en poids d'acide hyaluronique, et entre 0,001 et 1% en poids de peptide cationique antimicrobien, par rapport au poids total de la composition.

Les compositions suivant l'invention peuvent comprendre en outre un ou plusieurs actifs complémentaires renforçant ou complétant avantageusement l'activité de l'association d'acide hyaluronique et de peptide antimicrobien, et compatibles, c'est-à-dire non susceptibles de réagir les uns sur les autres ou de masquer ou limiter leurs effets respectifs.

Par exemple, elles peuvent contenir du chitosane, ou la chitine, qui, en se combinant à l'acide hyaluronique, présente un effet de réseau réticulé protecteur qui favorise la production endogène des facteurs de croissance kératinocytaire induisant l'activité fibroblastique, augmentant la production de collagène et accélérant la différenciation des fibroblastes en myofibroblastes. Elles peuvent aussi contenir une base sphingoïde, telle que la sphinganine et la phytosphin-gosine, qui optimise l'effet antibactérien et renforce l'effet anti-inflammatoire du peptide.

Divers actifs secondaires peuvent être avantageusement ajoutés à la composition et choisis parmi, par exemple, un agent cicatrisant, un anti-inflammatoire, un anti-infectieux et une vitamine telle que la vitamine A ou E. L'agent cicatrisant peut être par exemple un sel de zinc, un extrait de graine de caroube riche en oligogalactomannanes. L'anti-inflammatoire peut être un polysaccharide tel que Rhamnosoft^{®} ou Teflose^{®} (Solabia) qui inhibe l'adhésion cellulaire et limite les réactions inflammatoires, ou un extrait de gomme de Boswellia serrata (Soothex^{®}) qui agit par inhibition enzymatique de la synthèse des leukotriènes. L'anti-infectieux complémentaire peut être choisi parmi un silanol et un activateur de formation de peptide antimicrobien tel que le méthyl caproyl tyrosinate (Defensamide^{®}).

Les compositions selon la présente invention peuvent se présenter sous toutes les formes galéniques usuelles pour une application topique, notamment pour application topique externe. Elles peuvent se présenter par exemple sous forme de solutions aqueuses ou hydro-alcooliques, de lotions micellaires, de solutions pour pulvérisation, de shampooings, de dispersions, de sérums, de lingettes, de patchs, tulles ou pansements à libération contrôlée, de gels (aqueux, anhydres ou lipophiles), d'oléogels (gels lipidiques), de pommades, de suspensions, de dispersions vésiculaires ioniques ou non ioniques, d'émulsions liquides ou semi-liquides (par exemple un lait), solide ou semi-solide. Les émulsions peuvent être du type huile dans eau (H/ E) ou eau dans huile (E/ H), par exemple des gels ou des crèmes.

Les excipients et supports utilisables pour la préparation des compositions conformes à la présente invention sont ceux couramment utilisés dans les préparations à usage dermatologique, et choisis en fonction de la forme d'administration retenue. A titre d'exemple, on peut citer des émulsionnants, des épaississants, des gélifiants, des adoucissants, des conservateurs, des solubilisants, des agents de suspension ainsi que des bases lavantes et des parfums.

L'agent émulsionnant peut être choisi par exemple parmi des polymères carboxyvinyliques à haut poids moléculaire, des polysorbates tels que le Polysorbate 20^{®} ou le Tween 60^{®}, des esters de sorbitan et plus particulièrement un stéarate, un palmitate, ou un laurate de sorbitan tel que l'Arlacel^{®}. On peut encore utiliser comme agent émulsionnant un dérivé d'acide stéarique ou palmitique, par exemple le stéarate de polyéthylène glycol, le stéarate de glycérol, le stéarate de PEG 100® (par exemple Arlacel 165^{®}), un stéareth ou un cétéareth, un alcool gras tel qu'un alcool stéarylique, caprylique, ou cétéarylique, par exemple le Montanov 68^{®}, ou encore une silicone émulsionnable.

Les gélifiants et épaississants sont incorporés dans la composition pour en améliorer la fluidité. Ils peuvent être choisis par exemple parmi les poly-acrylamides du type Carbopol, des copolymères acrylate/acide acrylique comme l'Aculyn^{®}, des acrylates réticulés comme un Carbopol Ultrez^{®}, des dérivés de cellulose comme l'hydroxypropyl cellulose, ou les gommes naturelles comme la gomme xanthane et la gomme adragante.

Les agents hydratants et adoucissants utilisés dans la composition peuvent être choisis par exemple parmi le propylène glycol, la glycérine, le butylène glycol et le beurre de Karité, ainsi que les alcools gras. Un agent de suspension approprié est par exemple une argile telle que bentonite ou smectite.

On peut aussi ajouter avantageusement un agent facilitant la pénétration à travers l'épiderme, tel que la Cosmopérine.

Les compositions conformes à la présente invention sont préparées par les techniques usuelles, en fonction de la forme d'administration choisie, les quantités voulues d'acide hyaluronique, ou de ses sels, étant mélangées avec le peptide antimicrobien, et éventuellement la chitine ou le chitosane, et les supports et excipients, dans un milieu physiologiquement acceptable.

Par physiologiquement acceptable, au sens de la présente invention, on entend des supports et excipients d'un type couramment utilisé dans les compositions dermatologiques en médecine humaine, neutres vis-à-vis des principes actifs utilisés, ne présentant pas d'effet toxique et n'occasionnant aucun effet secondaire néfaste sur la peau.

Par exemple, dans le cas d'une crème, on peut procéder par dispersion d'une phase grasse dans une phase aqueuse pour obtenir une émulsion huile dans eau, ou inversement pour préparer une émulsion eau dans huile, les principes actifs étant dans l'une ou l'autre phase.

La composition de l'invention, par exemple sous forme de crème, est appliquée de préférence deux à trois fois par jour sur la zone de la peau nécessitant le traitement, pendant une période de temps pouvant aller de quelques jours à quatre semaines suivant la gravité de l'affection.

Les exemples suivants illustrent l'invention plus en détail sans en limiter la portée. Dans tous les exemples de compositions qui suivent, les parties sont exprimées en poids, sauf indication contraire.

### Exemple 1

On prépare un gel aqueux en mélangeant à température ambiante les composants dans l'ordre indiqué ci-après.

| | |
|---|---|
| Eau déminéralisée | 78,60 |
| Carbopol Ultrez 20 | 0,80 |
| Soude (solution aqueuse 32%) | 0,50 |
| Glycérine | 2,00 |
| Shield Bact Peptide® (solution à 0,1%) | 5,00 |
| Eau déminéralisée | 10,00 |
| Acide hyaluronique (PrimalHyal 450®) | 0,10 |
| Eau déminéralisée | 2,00 |
| Pentylène glycol | 1,00 |

Un gel aqueux ainsi composé peut être utilisé typiquement deux à trois fois par jour pendant plusieurs jours consécutifs en fonction de la gravité de l'affection traitée.

### Exemple 2

Par une technique classique, on prépare une lotion ayant la composition ci-après.

| | |
|---|---|
| Eau déminéralisée | 82,65 |
| Carbopol Ultrez 20 | 0,45 |
| Soude (solution aqueuse 32%) | 0,30 |
| Eau déminéralisée | 7,00 |
| Acide hyaluronique (PrimalHyal 450®) | 0,10 |
| Glycérine | 2,00 |
| Teflose® (Solabia) | 2,00 |
| Huile d'olive PEG-7 ester | 2,00 |
| Polysorbate 20 | 0,50 |
| Shield Bact Peptide® (solution à 0,1%) | 3,00 |

Cette lotion peut être utilisée une à deux fois par jour pendant quelques jours pour le traitement d'affections ophtalmiques telles qu'une blépharite.

### Exemple 3

On prépare une crème antimicrobienne par les techniques usuelles en mélangeant successivement les phases contenant les composants indiqués ci-après, les phases A et B étant mélangées à chaud (65-70°C), la phase C étant ensuite ajoutée à 50°C en mélangeant soigneusement, puis la phase D.

| Phase A | |
|---|---|
| Emulsionnant non ionique (Montanov 68) | 4,00 |
| Alcool myristylique | 2,00 |
| Alcool stéarylique | 0,50 |
| Cetiol RLF^{®} | 3,00 |
| Stéarate de glycéryle | 3,00 |
| Huile de tournesol oléique | 4,00 |
| Phase B | |
| Eau déminéralisée | 67.90 |
| Carbopol Ultrez 21^{®} | 0,45 |
| Propanediol | 2,00 |

| Phase C | |
|---|---|
| Glycérine | 2,00 |
| Shield Bact Peptide® (solution à 0,1%) | 6,00 |

| Phase D | |
|---|---|
| Soude (solution aqueuse 32%) | 0,05 |
| Eau déminéralisée | 5,00 |
| Acide hyaluronique (PrimalHyal 300®) | 0,10 |

Le pH de la composition est ajusté à 6,5 par addition de soude 32%.

Cette crème peut être utilisée en application sur les zones de la peau à traiter, une à deux fois par jour pendant une période de temps qui est déterminée par le praticien, et qui peut aller de une à trois semaines selon la nature et la gravité de l'affection traitée.

### Exemple 4

L'effet du choix de l'acide hyaluronique sur la production de β-défensines et la prolifération kératinocytaire a été mesuré comme suit.

| | Béta défensines | Prolifération kératinocytaire |
|---|---|---|
| AH hpm | 0 | NS |
| AH pmm 1 | ++++ | S |
| AH bpm 2 | + | NS |
| AH tbpm 3 | (+) | NS |

Dans le tableau ci-dessus, le signe "+" est représentatif de l'efficacité, de très faible (+) à très forte (++++). NS signifie "non significative".

L'acide hyaluronique (AH) utilisé est à haut poids moléculaire (hpm), à poids moléculaire moyen selon l'invention (pmm - environ 450 kDa), à bas poids moléculaire (bpm) ou à très bas poids moléculaire (tbpm).

### Production des β-défensines :

Des cultures de kératinocytes en confluence ont été traitées pendant 18h avec un AH pmm à 0,2%, ou un témoin positif (LPS d'Escherichia coli 5 µg/ml) ou avec :
- d'une part, un acide hyaluronique de haut poids moléculaire (AH hpm)
- d'autre part, des acides hyaluroniques de bas poids moléculaire (AH de 20kDa et AH de 10kDa).

La libération de HBD2 a été évaluée dans le surnageant à l'aide d'un kit ELISA. Les valeurs présentées sont des moyennes des résultats.

| | Témoin | LPS | AH pmm | AH bpm | AH tbpm |
|---|---|---|---|---|---|
| pg/ml | 11 | 43 | 105 | 20 | 18 |

### Prolifération kératinocytaire :

A J0, une plaie est effectuée sur une monocouche de kératinocytes (HaCaT) en confluence. Les cellules sont ensuite traitées à 37°C dans 2,5% de SVF (sérum de veau foetal) sans AH (condition de référence) ou avec des AH de poids moléculaires croissants (AH 50, AH 300, AH 1.000).

La largeur entre les deux bords de la plaie est mesurée en 3 endroits différents sur la même photo pour chaque traitement à J0 et J1. Chaque expérience est réalisée 5 fois de manière indépendante. Les moyennes sont traitées statistiquement (analyse de variances à un facteur suivi d'un test de Dunnett (SigmaStat).

Seul le AH pmm (AH 300) permet d'accélérer le processus de réparation cutanée via la prolifération des kératinocytes (HaCat).

### Stimulation des défenses bactériennes :

3 solutions sont préparées :
Solution A : AH pmm 0,10%
Solution B : hexapeptide 0,05%
Solution C : hexapeptide 0,05 % + AH pmm 0,10%

L'hexapeptide utilisé est le Shield Bact Peptide® disponible dans le commerce (Infinitec). L'acide hyaluronique (AH pmm) a un poids moléculaire moyen de 300 kDa.

Les protéines des biopsies de peaux humaines après traitement (Solutions A B C ou le milieu seul) sont extraites sous agitation douce pendant 2 heures dans une solution contenant 5% d'acide acétique et des inhibiteurs de protéases (0,02 mM PMSF, 2 ng/ml pepstatine et 2 ng/ml de leupeptine).

Les protéines solubles du surnageant sont séchées sous vide et homogénéisées dans une solution de 0,01% d'acide acétique.

10⁴ bactéries/ml de E. Coli (ATCC 4157) sont incubées en milieu de croissance logarithmique dans un tampon phosphate (pH 7,4) et un volume final 100 µl.

Cette suspension de E. Coli est mélangée avec 30 µg d'extraits protéiques et avec 10 µl de solution à 0,01% d'acide acétique contenant les inhibiteurs de protéases (témoin négatif) et les mélanges sont incubés pendant 120 minutes à 37°C.

A la fin de l'incubation, 10 µl sont prélevés et des dilutions à 1/10 et 1/100 sont ensemencées en triplicate dans des boîtes de Petri contenant du bouillon caséine-soja et sont incubées une nuit à 37°C.

Après ensemencement en boîte de Petri et incubation pendant une nuit à 37°C, le nombre d'UFC est déterminé pour chaque échantillon.

Le tableau des résultats ci-dessous montre que l'association d'hexapeptide antimicrobien couplé à l'acide palmitique avec l'acide hyaluronique de poids moléculaire moyen selon l'invention (Solution C), se montre la plus efficace, le pourcentage d'inhibition de la formation d'UFC étant de 100%.

| | Témoin | Sol. A | Sol. B | Sol. C |
|---|---|---|---|---|
| % inhibition | 40 | 55 | 77 | 100 |

### Exemple 5

Deux études cliniques ont été effectuées sur des chiens de races différentes et une étude clinique a été effectuée sur des patients, en dermatologie humaine.

### Première étude chez le chien :

Cinq chiens de races différentes âgés de 3 à 10 ans présentant une blépharo-conjonctivite infectieuse des 2 yeux ont été traités par des solutions préparées comme suit.
- solution A : une solution micellaire renfermant 0,10% de AH pmm et 0,03 % d'hexapeptide, suivant l'invention a été appliquée sur l'oeil droit
- solution B : une solution renfermant 0,03% d'hexapeptide a été appliquée sur l'oeil gauche

Le poids moléculaire moyen de l'acide hyaluronique de la composition est de 450 kDa et l'hexapeptide est le Shield Bact Peptide (Infinitec).

L'évolution clinique a été suivie à 4, 8, 12, 24, et 48 H. Les résultats sont reportés dans le tableau ci-après.

| évolution | Chien | Oeil droit | Oeeil gauche |
|---|---|---|---|
| 4H | 1 | + | + |
| 8H | 2 | ++ | +(+) |
| 12H | 3 | +++ | ++ |
| 24H | 4 | +++ | ++ |
| 48H | 5 | ++++ | +++ |

Les résultats montrent une amélioration plus rapide de la symptomatologie infectieuse avec la solution A suivant l'invention qui s'exprime dès la 8ème heure et se confirme à 12 et 24 heures

### Deuxième étude chez le chien :

Cas n° 1 : Chien Airedale terrier (10 ans) présentant une kérato-conjonctivite sèche évoluant depuis plusieurs années et traitée sans succès par la cyclosporine. L'oeil est sec, avec une conjonctivite surinfectée et réduction de la fente palpébrale. L'application de la lotion A ci-dessus une fois par jour pendant 8 jours entraîne une amélioration spectaculaire (oeil propre, ouvert, avec disparition de la douleur).
Cas n° 2 : Chien Bichon maltais (15 ans) présentant une ulcération cornéenne sévère à la suite d'une anesthésie de longue durée, sans amélioration par les traitements usuels. L'oeil reste sec, l'ulcération est profonde et la douleur est vive. L'utilisation de la lotion A suivant l'invention 2 fois par jour pendant 8 jours entraîne la cicatrisation et la disparition de la douleur.
Cas n° 3 : Chien Boxer West (7 ans) présentant un ulcère de la cornée avec tissu de granulation de 8 mm. La guérison est obtenue en utilisant la lotion A 2 fois par jour pendant 8 jours.
Cas n° 4 : Chien Tzu Bans présentant une kérato-conjonctivite sèche avec ulcération cornéenne et douleurs. Un traitement usuel pendant 3 semaines reste sans effet. L'utilisation de la lotion A suivant l'invention 2 fois par jour pendant 8 jours entraîne une atténuation sensible des douleurs sans modification nette de l'ulcération. Le passage à la cyclosporine entraîne une amélioration clinique de l'ulcération en 8 jours.
Cas n° 5 : chaton présentant un coryza sévère compliqué d'une perforation du globe oculaire. Une antibiothérapie n'apporte aucune amélioration. Echec d'une occlusion palpébrale thérapeutique. La lotion A suivant l'invention est alors utilisée 3 fois par jour et se traduit par une amélioration rapide.

### Etude chez l'homme :

La dermite atopique est caractérisée par une sensibilité particulière vis-à-vis du staphylocoque doré. Son rôle est souligné lors des poussées eczémateuses et le contrôle de sa prolifération à la surface de la peau est indispensable pour améliorer la symptomatologie.

Quatre patients adultes, 2 hommes et 2 femmes, âgés de 18 à 31 ans, présentant une poussée eczémateuse surinfectée siégeant au niveau des grands plis (plis du coude, creux poplités), ont été traités par deux émulsions fluides.
- émulsion A comportant l'hexapeptide seul à 0,05%, appliquée sur le coté droit ;
- émulsion B comportant l'hexapeptide (0,05 %) et le AH pmm (0,20%) appliquée sur le coté gauche.

Le poids moléculaire moyen de l'acide hyaluronique est de 300 kDa. L'hexapeptide est le Shield Bact Peptide (Infinitec).

Les critères Prurit, Suintement, Surinfection, ont été côtés de 0 à 4 de façon à définir un scoring d'évolution.

| | T0 | | T12 | | T24 | | T48 | | |
|---|---|---|---|---|---|---|---|---|---|
| SYMPTOMES | A | B | A | B | A | B | A | B | RESULTATS |
| PRURIT | 4 | 4 | 3 | 2 | 2 | 1 | 2 | 1 | B>A |
| SUINTEMENT | 4 | 4 | 2 | 1 | 1 | 0 | (1) | 0 | B>A |
| SURINFECTION | 4 | 4 | 2 | 1 | 1 | 0 | 0 | 0 | B>A |

Ces résultats montrent que les deux crèmes se montrent efficaces, mais que la crème B a une action plus rapide sur la symptomatologie se manifestant dès la 12ème heure.

La surinfection est maîtrisée à 24H

L'amélioration globale est plus nette avec la crème B suivant l'invention.

## Revendications

1. Composition dermatologique antimicrobienne topique pour utilisation en médecine humaine et vétérinaire, comprenant en association au moins un peptide antimicrobien cationique couplé à un lipide, et de l'acide hyaluronique de poids moléculaire compris entre 100 kDa et 800 kDa, ou un de ses sels, le peptide antimicrobien étant un hexapeptide couplé à l'acide palmitique comprenant des ponts disulfure.

2. Composition dermatologique selon la revendication 1, **caractérisée en ce que** l'acide hyaluronique est de poids moléculaire compris entre 200 kDa et 600 kDa.

3. Composition selon l'une quelconque des revendications précédentes, **caractérisée en ce que** l'acide hyaluronique est de l'acide hyaluronique hydrolysé.

4. Composition selon la revendication 1 ou 2, **caractérisée en ce que** le sel d'acide hyaluronique est choisi parmi les sels de métaux alcalins et alcalino-terreux.

5. Composition selon la revendication 4, **caractérisée en ce que** le sel d'acide hyaluronique est le hyaluronate de sodium, de potassium, de calcium ou de magnésium.

6. Composition selon l'une quelconque des revendications précédentes, **caractérisée en ce que** le peptide antimicrobien est couplé à un lipide par liaison covalente.

7. Composition selon la revendication 6, **caractérisée en ce que** le peptide antimicrobien est couplé par liaison covalente à un acide gras de 6 à 22 atomes de carbone.

8. Composition selon l'une quelconque des revendications précédentes, **caractérisée en ce qu'**elle comprend en outre de la chitine ou du chitosane.

9. Composition selon l'une quelconque des revendications précédentes, **caractérisée en ce qu'**elle comprend entre 0,005 et 2% en poids d'acide hyaluronique, et entre 0,001 et 1% en poids de peptide antimicrobien cationique, par rapport au poids total de la composition.

10. Composition selon l'une quelconque des revendications précédentes, **caractérisée en ce qu'**elle est présentée sous forme pour application topique externe.

11. Composition selon l'une quelconque des revendications précédentes, **caractérisée en ce qu'**elle comprend en outre un ou plusieurs actifs secondaires choisis parmi un agent cicatrisant, un anti-inflammatoire, un anti-infectieux et une vitamine telle que la vitamine A ou E.

## Patentansprüche

1. Topische antimikrobielle dermatologische Zubereitung für die Anwendung in der Human- oder Veterinärmedizin, umfassend in Kombination mindestens ein kationisches antimikrobielles Peptid, gekoppelt an ein Lipid, und Hyaluronsäure mit Molekulargewicht zwischen 100 kDa und 800 kDa inklusive, oder eines ihrer Salze, wobei das antimikrobielle Peptid ein Hexapeptid ist, das an Palmitinsäure gekoppelt ist, umfassend Disulfidbrücken.

2. Dermatologische Zubereitung nach Anspruch 1, **dadurch gekennzeichnet, dass** die Hyaluronsäure ein Molekulargewicht zwischen 200 kDa und 600 kDa inklusive hat.

3. Zubereitung nach einem der vorangehenden Ansprüche, **dadurch gekennzeichnet, dass** die Hyaluronsäure hydrolysierte Hyaluronsäure ist.

4. Zubereitung nach Anspruch 1 oder 2, **dadurch gekennzeichnet, dass** das Salz der Hyaluronsäure aus den Salzen alkalischer und erdalkalischer Metalle ausgewählt ist.

5. Zubereitung nach Anspruch 4, **dadurch gekennzeichnet, dass** das Salz der Hyaluronsäure das Natrium-, Kalium-, Kalzium- oder Magnesiumhyaluronat ist.

6. Zubereitung nach einem der vorangehenden Ansprüche, **dadurch gekennzeichnet, dass** das antimikrobielle Peptid mittels kovalenter Bindung an ein Lipid gekoppelt ist.

7. Zubereitung nach Anspruch 6, **dadurch gekennzeichnet, dass** das antimikrobielle Peptid mittels kovalenter Bindung an eine Fettsäure mit 6 bis 22 Kohlenstoffatomen gekoppelt ist.

8. Zubereitung nach einem der vorangehenden Ansprüche, **dadurch gekennzeichnet, dass** sie ferner Chitin oder Chitosan umfasst.

9. Zubereitung nach einem der vorangehenden Ansprüche, **dadurch gekennzeichnet, dass** sie zwischen 0,005 und 2 Gew.-% Hyaluronsäure und zwischen 0,001 und 1 Gew.-% kationisches antimikrobielles Peptid im Verhältnis zum Gesamtgewicht der Zubereitung umfasst.

10. Zubereitung nach einem der vorangehenden Ansprüche, **dadurch gekennzeichnet, dass** sie in Form für äußere topische Anwendung präsentiert ist.

11. Zubereitung nach einem der vorangehenden Ansprüche, **dadurch gekennzeichnet, dass** sie ferner einen oder mehrere sekundäre Wirkstoffe umfasst, ausgewählt aus einem Heil-, Antientzündungs-, Antiinfektionsmittel und einem Vitamin wie das Vitamin A oder E.

## Claims

1. A topical antimicrobial dermatological composition for use in human and veterinary medicine, comprising in an association at least one cationic antimicrobial peptide coupled with a lipid, and hyaluronic acid with a molecular weight comprised between 100 kDa and 800 kDa, or one of its salts, the antimicrobial peptide being a hexapeptide coupled with palmitic acid comprising disulfide bridges.

2. The dermatological composition according to claim 1, **characterized in that** the hyaluronic acid is of a molecular weight comprised between 200 kDa and 600 kDa.

3. The composition according to any of the preceding claims, **characterized in that** the hyaluronic acid is hydrolyzed hyaluronic acid.

4. The composition according to claim 1 or 2, **characterized in that** the hyaluronic acid salt is selected from among alkaline and earth alkaline metal salts.

5. The composition according to claim 4, **characterized in that** the hyaluronic acid salt is sodium, potassium, calcium or magnesium hyaluronate.

6. The composition according to any of the preceding claims, **characterized in that** the antimicrobial peptide is coupled to a lipid through a covalent bond.

7. The composition according to claim 6, **characterized in that** the antimicrobial peptide is coupled through a covalent bond to a fatty acid with 6 to 22 carbon atoms.

8. The composition according to any of the preceding claims, **characterized in that** it further comprises chitin or chitosan.

9. The composition according to any of the preceding claims **characterized in that** it comprises between 0.005 and 2% by weight of hyaluronic acid, and between 0.001 and 1% by weight of a cationic antimicrobial peptide, are based on the total weight of the composition.

10. The composition according to any of the preceding claims, **characterized in that** it is shown as a form for external topical application.

11. The composition according to any of the preceding claims, **characterized in that** it further comprises one or several secondary actives are selected from among a healing agent, an anti-inflammatory agent, an anti-infectious agent and a vitamin such as vitamin A or E.
